# EUROPÄISCHE PATENTSCHRIFT

(11) **EP 3 576 804 B1**
(45) Veröffentlichungstag und Bekanntmachung des Hinweises auf die Patenterteilung: **19.03.2025**
(21) Anmeldenummer: 17812253.7
(22) Anmeldetag: 08.11.2017
(51) Int. Cl.: A61M 60/113, A61M 60/268, A61M 60/38, A61M 60/427, A61M 60/851

(54) **EXTRAKORPORALE BLUTPUMPE, HERZ-LUNGEN-MASCHINE, VERFAHREN ZUM BETREIBEN EINER EXTRAKORPORALEN BLUTPUMPE SOWIE VERFAHREN ZUM BETREIBEN EINER HERZ-LUNGEN-MASCHINE**
EXTRACORPOREAL BLOOD PUMP, HEART-LUNG MACHINE, METHOD FOR OPERATING AN EXTRACORPOREAL BLOOD PUMP, AND METHOD FOR OPERATING A HEART-LUNG MACHINE
POMPE À SANG EXTRACORPORELLE, MACHINE COEUR-POUMON, PROCÉDÉ POUR FAIRE FONCTIONNER UNE POMPE À SANG EXTRACORPORELLE AINSI QUE PROCÉDÉ POUR FAIRE FONCTIONNER UNE MACHINE COEUR-POUMON

(30) Priorität: 31.01.2017 DE 102017000843
(43) Veröffentlichungstag der Anmeldung: 11.12.2019
(73) Patentinhaber: Hemovent GmbH, 52076 Aachen (DE)
(72) Erfinder: HUTZENLAUB, Jens, 52066 Aachen (DE)
(74) Vertreter: Sattler de Sousa e Brito, Clara
(86) Internationale Anmeldenummer: PCT/DE2017/000376
(87) Internationale Veröffentlichungsnummer: WO 2018/141316

(56) Entgegenhaltungen:
- EP-A1- 3 520 833
- EP-A2- 2 517 739
- WO-A2-02/30267
- US-A- 3 916 449
- US-A- 5 232 434
- US-A1- 2007 158 247

## Beschreibung

Die Erfindung betrifft eine extrakorporale Blutpumpe, eine Herz-Lungen-Maschine, ein Verfahren zum Betreiben einer extrakorporalen Blutpumpe sowie ein Verfahren zum Betreiben einer Herz-Lungen-Maschine.

Das Herz als zentrales Organ des Kreislaufsystems ist ein Hohlmuskel mit zwei Kammern, der durch Kontraktion und Erschlaffen das Blut im Kreislauf fördert. Mit seiner linken Kammer (linker Ventrikel) wird das Blut durch die arteriellen Blutgefäße des großen Kreislaufs zu den Blutkapillaren der Körperperipherie gepumpt. Durch die venösen Blutgefäße gelangt das Blut zurück zur rechten Herzkammer (rechter Ventrikel). Von dort wird es durch die Lungenarterien in den Lungenkreislauf (kleiner Kreislauf) zur Lunge gefördert und gelangt über die Lungenvenen wieder zurück zur linken Herzkammer. Der kleine Kreislauf befindend sich im Brustkorb.

Bei Herzerkrankungen können Patienten in eine Situation gelangen, in der eine künstliche Kreislaufunterstützung zur einzig möglichen und damit lebenserhaltenden Therapie wird.

Herz-Lungen-Maschinen ersetzen die lebenswichtigen Kreislauffunktionen der Blutförderung und des Gasaustauschs beispielsweise während einer Herzoperation. Abgeleitet davon können Herz-Lungen-Maschinen auch eingesetzt werden, um Patient mit Herz- oder Lungeninsuffizienz über Tage zu stabilisieren. Hierbei spricht man von Extrakorporaler Membranoxygenierung (ECMO) oder Extrakorporalen Life Support (ECLS). Hierbei wird das Blut über Kanülen, die minimalinvasiv eingebracht werden können, dem Patienten entnommen, aufbereitet und wieder in den Patienten zurückgeführt.

Die WO 2009/024308 A1 offenbart einen elektrischen Linearantrieb, insbesondere für ein Pumpsystem von einem Kunstherz.

Die EP 2 523 702 A1 offenbart eine Anordnung mit einer Blutpumpe und einem Gasaustauscher zur extrakorporalen Membranoxygenierung.

Das Dokument WO02/30267 offenbart eine extrakorporale Blutpumpe zum Ansangen und Verdrängen von Blut, wobei die Blutpumpe zwei Blutkammern und eine mechanische Antriebseinheit aufweist, wobei die Antriebseinheit zwischen den Blutkammern angeordnet ist, wobei eine Blutkammer eine Membran, einen Bluteintrittskanal und einen Blutaustrittskanal aufweist, dadurch gekennzeichnet, dass die Blutaustrittskanäle beider Blutkammern miteinander verbunden sind.

Das Dokument US5232434 offenbart eine extrakorporale Blutpumpe zum Ansaugen und Verdrängen von Blut, wobei die Blutpumpe eine Blutkammer und eine mechanische Antriebseinheit aufweist, wobei die Antriebseinheit an die Blutkammer angrenzt, wobei die Blutkammer eine Membran, einen Bluteintrittskanal und einen Blutaustrittskanal aufweist, wobei die Antriebseinheit einem Gaseinlass und einem Gasauslass sowie zwei Druckkammern aufweist, wobei jede Druckkammer eine Wirkfläche aufweist, insbesondere ist eine Wirkfläche eine Membran, wobei eine Blutkammer über eine Membran von einer Druckkammer getrennt ist, wobei die Antriebseinheit in Wirkverbindung zur Lage der Membran steht.

Das Dokument EP3520833, Stand der Technik gemäß Artikel 54(3) EPÜ, offenbart eine extrakorporale Blutpumpe zum Ansaugen und Verdrängen von Blut, wobei die Blutpumpe zwei Blutkammern und eine mechanische Antriebseinheit aufweist, wobei jede der Blutkammern eine Membran, einen Bluteintrittskanal und einen Blutaustrittskanal aufweist,- wobei die Antriebseinheit zwischen den Blutkammern angeordnet ist, die Blutaustrittskanäle beider Blutkammern miteinander verbunden sind und ein Bereich zwischen den Membranen eine Koppelstange aufweist, welche dazu eingerichtet ist, die Bewegung der zwei Membranen miteinander zu koppeln.

Der Erfindung liegt die Aufgabe zugrunde, dem Stand der Technik eine Verbesserung oder eine Alternative zur Verfügung zu stellen.

Nach einem ersten Aspekt der Erfindung löst die Aufgabe eine extrakorporale Blutpumpe zum Ansaugen und Verdrängen von Blut, wobei die Blutpumpe zwei Blutkammern und eine mechanische Antriebseinheit aufweist, wobei die Antriebseinheit zwischen den Blutkammern angeordnet ist, wobei eine Blutkammer eine Membran, einen Bluteintrittskanal und einen Blutaustrittskanal aufweist, wobei die Blutaustrittskanäle beider Blutkammern miteinander verbunden sind.

Begrifflich sei hierzu Folgendes erläutert:
Zunächst sei ausdrücklich darauf hingewiesen, dass im Rahmen der hier vorliegenden Patentanmeldung unbestimmte Artikel und Zahlenangaben wie "ein", "zwei" usw. im Regelfall als "mindestens"-Angaben zu verstehen sein sollen, also als "mindestens ein...", "mindestens zwei ..." usw., sofern sich nicht aus dem jeweiligen Kontext ausdrücklich ergibt oder es für den Fachmann offensichtlich oder technisch zwingend ist, dass dort nur "genau ein ...", "genau zwei ..." usw. gemeint sein können.

Eine "Blutpumpe", oft auch als "Kunstherz" bezeichnet, ist ein Gerät, welches die Funktion des Herzens übernimmt oder unterstützt und dazu eingerichtet ist den Kreislauf des Körpers aufrechtzuerhalten. Insbesondere kann eine Blutpumpe zur Unterstützung im Rahmen einer Operation, insbesondere zur temporären Übernahme der Funktion des Herzens oder zur Unterstützung des Herzens (perioperativ), zur Übernahme der Funktion des Herzens oder zur Unterstützung des Herzens nach einer Operation (postoperativ) oder anderweitig also nicht in Verbindung mit dem Ereignis einer Operation im Rahmen einer Intervention zum Einsatz kommen.

Eine "extrakorporale Blutpumpe" wird außerhalb des Körpers eingesetzt.

Eine "Blutkammer" ist ein Bauteil einer Blutpumpe und ist dazu eingerichtet von dem designierten Blutstrom durchströmt zu werden. Eine Blutkammer weist eine "Membran" auf.

Eine "Membran" ist ein Bauteil einer Blutpumpe sowie eine Komponente einer Blutkammer und ist dazu eingerichtet ihre Lage und damit das Volumen der Blutkammer zu verändern, wobei der designierte Blutstrom bei einem Vergrößern des Blutkammervolumens angesaugt und bei einem Reduzieren des Blutkammervolumens verdrängt wird. Insbesondere ist die "Membran" dazu eingerichtet ihre Lage so verändern zu können, dass eine Blutkammer nur noch etwa 10 % oder weniger ihres maximalen Volumens aufweisen kann, ohne dass es dabei zu Beeinträchtigungen im Materialverhalten der Blutkammer oder der Membran kommt.

Eine "Antriebseinheit", insbesondere eine "mechanische Antriebseinheit", ist eine konstruktive Einheit, die mittels Energieumformung eine Blutpumpe antreibt.

Ein "Bluteintrittskanal" ist ein Kanal durch den der designierte Blutstrom einer sich im Betrieb befindlichen Blutpumpe in eine einzelne Blutkammer einströmt.

Ein "Blutaustrittskanal" ist ein Kanal durch den der designierte Blutstrom einer sich im Betrieb befindlichen Blutpumpe aus einer einzelne Blutkammer ausströmt.

Der Stand der Technik sah bislang vor, dass zur Unterstützung der Funktion des Herzens oder zur Übernahme der Funktion des Herzens vorwiegend Kreiselpumpen zum Einsatz kommen. Kreiselpumpen können das Blut im Betrieb bauformbedingt mechanisch durch ein vergleichsweise hohes auf das Blut einwirkendes Scherspannungsniveau im Blut belasten.

Im praktischen Betrieb von Kreiselpumpen kann es häufig zum Ansaugen von Kanülen auf der Einlassseite der Kreiselpumpe kommen. Durch die Druck-Fluss-Charakteristik von Kreiselpumpen kann sich inhärent der Ansaugdruck verstärken und es kann zu einer weiteren Erhöhung der auf das Blut einwirkenden Scherspannung oder gar zur Kavitation des Blutes führen, wodurch es zu einer Blutschädigung kommen kann.

Alternativ werden im Stand der Technik Membranpumpen eingesetzt.

Abweichend wird eine spezifische Bauform einer Membranpumpe vorgeschlagen, welche zwei Blutkammern und eine mechanische Antriebseinheit aufweist, wobei die Antriebseinheit zwischen den Blutkammern angeordnet ist, wobei eine Blutkammer eine Membran, einen Bluteintrittskanal und einen Blutaustrittskanal aufweist, wobei die Blutaustrittskanäle beider Blutkammern miteinander verbunden sind.

Es ist konkret unter anderen Konstruktionsweisen denkbar, dass die zwei Blutkammern das gleiche nominale Volumen aufweisen.

Bei einer geeigneten Gestaltung der Blutpumpe werden Hinterschnitte und Totwassergebiete im vom designierten Blutstrom durchströmten Bereich vermieden.

Vorteilhaft kann durch den hier vorgestellten Aspekt der Erfindung erreicht werden, dass die blutführende Seite der Blutpumpe so gestaltet werden kann, dass der Blutstrom möglichst blutschonend geführt werden kann.

Damit kann vorteilhaft erreicht werden, dass die auf das Blut wirkenden Kräfte vergleichsweise gering bleiben und alle Bereiche der Blutpumpe von dem Blut stets ausgewaschen werden.

Somit kann vorteilhaft verhindert werden, dass Blutzellen geschädigt werden und das Blut verklumpt.

Vorteilhaft kann hierdurch eine besonders blutschonende und energetisch effiziente Blutpumpe erreicht werden, welche den designierten Blutstrom durch eine Kanüle in den Körper zurückfließen lassen kann.

Bevorzugt sind die Bluteintrittskanäle beider Blutkammern miteinander verbunden.

In einer geeigneten Ausführungsform sind die beiden Bluteintrittskanäle in einer Y-Anordnung miteinander verbunden, so dass der designierte Blutstrom auf beide Blutkammern aufgeteilt werden kann.

Vorteilhaft kann hierdurch erreicht werden, dass das Blut mit nur einer Kanüle dem Körper entnommen werden kann und dass der designierte Blutstrom besonders blutschonend in der Blutpumpe bedruckt wird.

Optional weist ein Verbindungsbereich der Blutaustrittskanäle eine Rückströmdrossel auf.

Begrifflich sei hierzu Folgendes erläutert:
Ein "Verbindungsbereich" ist ein Bereich in dem die designierten Teilströme des Blutes aus beiden Blutkammern wieder zusammenfließen.

Eine "Rückströmdrossel" bezeichnet ein Bauteil, welches dazu eingerichtet ist in einer Strömungsrichtung mit einem geringen Druckverlust und in der entgegengesetzten Strömungsrichtung mit einem großen Druckverlust durchströmt zu werden. Insbesondere ist der Druckverlust in Rückstromrichtung so hoch, dass ein Rückströmen verhindert werden kann. Bevorzugt stellt eine Rückströmdrossel einen Verschluss für einen designierten Blutstrom in einer Strömungsrichtung dar, während ein designierter Blutstrom die Rückströmdrossel in der anderen Strömungsrichtung beinahe ungehindert passieren kann, insbesondere ungehindert passieren kann. In gewisser Weise weist eine Rückströmdrossel die Eigenschaften eines verstellbareren Verschlusses auf, welcher in Abhängigkeit der designierten Strömungsrichtung geöffnet oder verschlossen wird.

So ist konkret unter anderem denkbar, dass die Blutaustrittskanäle in einem Verbindungsbereich von beiden Seiten ähnlich einer sogenannten Y-Anordnung zusammengeführt werden.

Damit wird bei einer geeigneten Gestaltung erreicht, dass beim Auswerfen eines designierten Blutstroms ein Rückströmen des Bluts in die andere Blutkammer verhindert wird. In einer bevorzugten Ausführungsform ist die Rückströmdrossel so gestaltet, dass sie von dem designierten Blutstrom auf beiden Seiten gut umströmt wird, wodurch die Rückströmdrossel stets gut ausgewaschen wird.

Vorteilhaft kann hierdurch erreicht werden, dass die Blutpumpe eine hohe energetische Effizienz aufweist.

Außerdem kann vorteilhaft erreicht werden, dass die Rückströmdrossel sowie der Verbindungsbereich von einem designierten Blutstrom gut ausgewaschen werden und so eine Bildung von Thromben verhindert werden kann.

Weiterhin kann vorteilhaft erreicht werden, dass beim Ansaugen Blut über den Blutaustrittskanal in die Blutkammer gelangen kann.

Erfindungsgemäß weist die Antriebseinheit einen Gaseinlass und einen Gasauslass sowie eine Druckkammer auf, wobei die Druckkammer über eine Membran von einer Blutkammer getrennt ist, wobei die Antriebseinheit in Wirkverbindung zur Lage der Membran steht.

Begrifflich sei hierzu Folgendes erläutert:
Ein "Gaseinlass" ist ein Einlass für ein gasförmiges Fluid. Der Gaseinlass wird von einem designierten Gasstrom so durchströmt, dass Gas in die Antriebseinheit einströmt.

Ein "Gasauslass" ist ein Auslass für ein gasförmiges Fluid. Der Gasauslass wird von einem designierten Gasstrom so durchströmt, dass Gas aus der Antriebseinheit ausströmt.

Eine "Druckkammer" ist eine Kammer welche dazu eingerichtet ist von einem designierten Gasstrom durchströmt zu werden. Eine Druckkammer weist eine "Wirkfläche" auf. Die "Wirkfläche" ist dazu eingerichtet ihre Lage verändern zu können, wodurch Energie gewandelt werden kann. Insbesondere kann eine Wirkfläche eine Membran sein, die eine Blutkammer und eine Druckkammer voneinander trennt.

So ist konkret unter anderem denkbar, dass die Blutpumpe mit Gas betrieben wird, welches in der Antriebseinheit Energie an den Blutstrom abgibt.

In geeigneter Weise kann der Gasstrom von einer Versorgungseinheit zur Verfügung gestellt werden.

Erfindungsgemäß ist eine Druckkammer angrenzend an eine Blutkammer angeordnet. Dabei sind Druckkammer und Blutkammer lediglich durch die Membran voneinander getrennt.

So kann die Antriebseinheit in Wirkverbindung zur Lage der Membran stehen. Insbesondere kann durch ein Erhöhen des Druckes in der Druckkammer die Lage der Membran in Richtung der Blutkammer verschoben werden.

Insbesondere kann durch ein Reduzieren des Druckes in der Druckkammer die Lage der Membran in Richtung der Druckkammer verschoben werden.

In einer geeigneten Gestaltung der Antriebseinheit weist diese eine Wirkverbindung zur Lage der Membran auf, welche eine Endlagenerkennung der Membran einbezieht.

So ist es der Antriebseinheit in dieser geeigneten Ausführungsform möglich zu erkennen, wenn die Membran ihre jeweilige Endlage erreicht hat und ihre Bewegung gestoppt oder umgekehrt werden sollte.

Vorteilhaft kann hierdurch erreicht werden, dass die Antriebseinheit die notwendige Energie zum Ansaugen und Verdrängen des Blutes von einem Gasstrom beziehen kann.

Weiterhin kann vorteilhaft erreicht werden, dass die Wirkverbindung zur Lage der Membran eine Beschädigung der Membran verhindert, ein energieoptimales Ansaugen und Verdrängen des Bluts ermöglicht und zu einer möglichst blutschonenden Behandlung des Blutes in der Blutpumpe beiträgt.

Vorteilhaft kann außerdem erreicht werden, dass ein Ansaugen der Kanülen in den Blutgefäßen als ein häufiges Problem beim Einsatz von Blutpumpen verhindert werden kann, da das beschriebenen System durch die pneumatisch betriebene Antriebseinheit eine Ansauglimitierung aufweist, die durch eine Beziehung zwischen dem Druck des Betriebsgases, des Drucks auf der Auswurfseite und dem Ansaugdruck der Einlassseite definiert ist. So kann auf die aufwendigen Alarme und Sicherheitseinrichtung herkömmlicher System mittels einer Drucksensorik verzichtet werden.

Optional weist die Antriebseinheit zwei Druckkammern auf, wobei jede Druckkammer über eine Membran an eine Blutkammer angrenzt.

In einer bevorzugten Ausführungsform wird ein designierter Blutstrom in einer Blutkammer ausgeworfen, während er auf der anderen Seite angesaugt wird.

Bei geeigneter Gestaltung der Blutpumpe wird der Gasstrom wechselseitig jeweils eine Membran mit Druck beaufschlagen.

So ist konkret unter anderem denkbar, dass nach einem Arbeitsgang und/oder einem Erreichen einer Membranendlage der Gasdruck in der Druckkammer reduziert wird und die andere Druckkammer mit Gasdruck beaufschlagt wird, sodass sich beide Membranen nun in entgegengesetzter Richtung bewegen.

Vorteilhaft kann hierdurch erreicht werden, dass die Blutpumpe mit einem konstanten Gasstrom betrieben werden kann, wodurch keine externe Beschaltung notwendig ist.

Weiterhin kann vorteilhaft erreicht werden, dass ein designierter Gasstrom nur ein Druckniveau aufweisen muss.

Nach einem zweiten Aspekt der Erfindung löst die Aufgabe eine extrakorporale Blutpumpe zum Ansaugen und Verdrängen von Blut, wobei die Blutpumpe eine Blutkammer und eine mechanische Antriebseinheit aufweist, wobei die Antriebseinheit an die Blutkammer angrenzt, wobei die Blutkammer eine Membran, einen Bluteintrittskanal und einen Blutaustrittskanal aufweist, wobei die Antriebseinheit einem Gaseinlass und einem Gasauslass sowie zwei Druckkammern aufweist, wobei jede Druckkammer eine Wirkfläche aufweist, insbesondere ist eine Wirkfläche eine Membran, wobei eine Blutkammer über eine Membran von einer Druckkammer getrennt ist, wobei die Antriebseinheit in Wirkverbindung zur Lage der Membran steht.

Der Stand der Technik sah bislang vor, dass zur Unterstützung der Funktion des Herzens oder zur Übernahme der Funktion des Herzens vorwiegend Kreiselpumpen zum Einsatz kommen. Kreiselpumpen können das Blut im Betrieb bauformbedingt mechanisch durch ein vergleichsweise hohes auf das Blut einwirkendes Scherspannungsniveau im Blut belasten.

Alternativ werden im Stand der Technik Membranpumpen eingesetzt.

Abweichend wird eine spezifische Bauform einer Membranpumpe vorgeschlagen, welche eine Blutkammer und eine Antriebseinheit aufweist, wobei die Antriebseinheit zwei Druckkammern aufweist. Eine Druckkammer ist über die Membran mit der Blutkammer verbunden. Die andere Druckkammer ist seitlich neben der ersten Druckkammer positioniert.

In einer geeigneten Ausführungsform erlaubt die Blutpumpe ein zyklisches Ansaugen und Verdrängen von Blut mit der Blutkammer, wobei die Antriebseinheit entsprechend dem ersten Aspekt der Erfindung betrieben werden kann.

Bei einer geeigneten Gestaltung weist die zweite Druckkammer statt der nach dem ersten Aspekt eingesetzten Membran eine andersartige Wirkfläche auf.

Vorteilhaft kann hierdurch erreicht werden, dass die Blutpumpe mit einer Blutkammer besonderes blutschonend betrieben werden kann und die Antriebseinheit die notwendige Energie zum Ansaugen und Verdrängen des Blutes von einem Gasstrom beziehen kann.

Weiterhin kann vorteilhaft erreicht werden, dass die Wirkverbindung zur Lage der Membran eine Beschädigung der Membran verhindert, ein energieoptimales Ansaugen und Verdrängen des Bluts ermöglicht und zu einer möglichst blutschonenden Behandlung des Blutes in der Blutpumpe beiträgt.

Vorteilhaft kann außerdem erreicht werden, dass ein Ansaugen der Kanülen in den Blutgefäßen als ein häufiges Problem beim Einsatz von Blutpumpen verhindert werden kann, da das beschriebenen System durch die pneumatisch betriebene Antriebseinheit eine Ansauglimitierung aufweist, die durch eine Beziehung zwischen dem Druck des Betriebsgases, des Drucks auf der Auswurfseite und dem Ansaugdruck der Einlassseite definiert ist. So kann auf die aufwendigen Alarme und Sicherheitseinrichtung herkömmlicher System mittels einer Drucksensorik verzichtet werden.

Vorteilhaft kann außerdem hierdurch erreicht werden, dass die Blutpumpe mit einem konstanten Gasstrom betrieben werden kann, wodurch keine externe Beschaltung notwendig ist.

Weiterhin kann vorteilhaft erreicht werden, dass ein designierter Gasstrom nur ein Druckniveau aufweisen muss.

Bevorzugt weist eine Blutkammer einen rotationssymmetrischen Bereich auf.

Vorteilhaft kann hierdurch erreicht werden, dass die von der Geometrie der Blutkammer auf den designierten Blutstrom einwirkenden Kräfte möglichst ein geringes homogenes Niveau erreichen können.

Weiterhin kann vorteilhaft erreicht werden, dass das Strömungsfeld im Inneren einer Blutkammer möglichst blutschonend gestaltet werden kann.

Vorteilhaft kann hiermit auch erreicht werden, dass die Blutpumpe eine hohe energetische Effizienz aufweist.

Optional sind ein Bluteintrittskanal und ein Blutaustrittskanal vorwiegend in Umfangsrichtung an den rotationssymmetrischen Bereich einer Blutkammer angeordnet.

Damit kann vorteilhaft erreicht werden, dass die auf das Blut wirkenden Kräfte vergleichsweise gering bleiben und alle Bereiche der Blutpumpe von dem Blut stets ausgewaschen werden.

Somit kann vorteilhaft verhindert werden, dass Blutzellen geschädigt werden und das Blut verklumpt.

Vorteilhaft kann hierdurch eine besonders blutschonende und energetisch effiziente Blutpumpe erreicht werden.

Bevorzugt weist ein Bluteintrittskanal eine Rückströmdrossel auf.

In einer vorteilhaften Ausführungsform weist der Bluteintrittskanal eine Rückströmdrossel auf, der dazu eingerichtet ist ein Strömen von Blut entgegen der designierten Strömungsrichtung des Bluts zu verhindern.

Besonders bevorzugt ist die Rückströmdrossel so gestaltet, dass sie von dem designierten Blutstrom besonders gut ausgewaschen wird.

Vorteilhaft kann hierdurch erreicht werden, dass Blut beim Verdrängen in einer Blutkammer über den Bluteintrittskanal strömt.

Außerdem kann vorteilhaft verhindert werden, dass sich an der Rückströmdrossel Thromben bilden.

Vorteilhaft kann hierdurch weiterhin erreicht werden, dass die Blutpumpe eine hohe energetische Effizienz aufweist.

Optional weist die Antriebseinheit ein Gaseinlassventil und ein Gasauslassventil sowie eine Umschaltvorrichtung auf, wobei das Gaseinlassventil und das Gasauslassventil gegenüber einem Gasströmweg eine Verschlussstellung und eine Öffnungsstellung aufweisen, wobei die Umschaltvorrichtung zwei Schaltendzustände aufweist, insbesondere weist die Umschaltvorrichtung genau zwei Schaltzustände auf.

Begrifflich sei hierzu Folgendes erläutert:
Ein "Gaseinlassventil" ist ein Bauteil zur Absperrung oder Regelung des Durchflusses von Gas. Im Gaseinlassventil wird ein Verschlussteil bewegt, wodurch die Strömung reduziert oder unterbrochen wird. Insbesondere ist das Gaseinlassventil dazu eingerichtet Gas in die Druckkammer einströmen zu lassen.

Ein "Gasauslassventil" ist ein Bauteil zur Absperrung oder Regelung des Durchflusses von Gas. Im Gasauslassventil wird ein Verschlussteil bewegt, wodurch die Strömung reduziert oder unterbrochen wird. Insbesondere ist das Gasauslassventil dazu eingerichtet Gas in die Druckkammer ausströmen zu lassen.

Eine "Umschaltvorrichtung" ist eine mechanische Baugruppe, die einen anderen Schaltzustand herbeiführen kann. Die Umschaltvorrichtung schaltet kurz vor dem Erreichen der Membranendlage das Gaseinlassventil und das Gasauslassventil um, wodurch die jeweils andere Druckkammer bedruckt wird.

Unter "Bistabilität" wird die Eigenschaft eines Systems verstanden, zwei mögliche stabile Zustände einnehmen zu können, aber nur durch einen äußeren Impuls vom einen in den anderen Zustand zu wechseln. Diese Systeme heißen dann bistabile Systeme.

Eine "Membranendlage" oder "Wirkflächenendlage" bezeichnet den designierten Umkehrpunkt einer Membranlage oder Wirkflächenlage.

Insbesondere ist die Umschaltvorrichtung bistabil ausgeführt und kann so nur diskrete Positionen in den jeweiligen Endlagen einnehmen.

Ein "Gasströmweg" ist ein Weg den ein designierter Gasstrom nimmt.

Eine "Verschlussstellung" ist die Stellung eines Ventils, insbesondere eines Gaseinlassventils und/oder eines Gasauslassventils, bei der ein Strömen von Gas verhindert ist.

Eine "Öffnungsstellung" ist die Stellung eines Ventils, insbesondere eines Gaseinlassventils und/oder eines Gasauslassventils, bei der ein Strömen von Gas möglich ist.

Ein "Schaltendzustand" ist ein stabiler Schaltzustand, der nach einem abgeschlossenen Schaltzustand eines bistabilen Schaltsystems erreicht wird.

In einer besonders bevorzugten Ausführungsform ist die Umschaltvorrichtung derart gestaltet, dass sich die Umschaltvorrichtung beim Erreichen einer Membranendlage zwischen den beiden möglichen Schaltendzuständen umschaltet und so beim Betätigen der Umschaltvorrichtung die Ventilstellungen in den jeweils anderen Schaltendzustand übergeht, wodurch beim Erreichen einer Membranendlage der Gasdruck in der Druckkammer reduziert wird und die andere Druckkammer mit Gasdruck beaufschlagt wird, sodass sich beide Membranen oder Wirkflächen nun in entgegengesetzter Richtung bewegen.

So kann bei einer geeigneten Ausführungsform die Membranendlage insbesondere erreicht werden, wenn eine Blutkammer nur noch etwa 10 % oder weniger ihres maximalen Volumens aufweist.

So können sich die verbundenen Wirkflächen und/oder Membranen nach einem Arbeitsgang in die gegenüberliegende Richtung bewegen, indem die mit Druck beaufschlagte Druckkammer durch ein Umschalten durch die Umschaltvorrichtung gewechselt wird.

Vorteilhaft kann hierdurch erreicht werden, dass eine Blutpumpe erreicht wird, welche mechanisch autonom arbeitet und dabei nicht auf eine Elektronik oder äußere Vorgaben angewiesen ist.

Weiterhin kann vorteilhaft erreicht werden, dass es nicht zu einer Beeinträchtigung im Materialverhalten der Blutkammer oder der Membran kommt.

Vorteilhaft kann hierdurch außerdem erreicht werden, dass die Blutpumpe mit einem konstanten Gasstrom betrieben werden kann, wodurch keine externe Beschaltung notwendig ist.

Bevorzugt weist ein Schaltendzustand für eine Blutkammer das Gaseinlassventil in Öffnungsstellung und das Gasauslassventil in Verschlussstellung oder das Gaseinlassventil in Verschlussstellung und das Gasauslassventil in Öffnungsstellung auf.

Vorteilhaft kann hierdurch erreicht werden, dass jeweils eine Druckkammer mit Gas gefüllt wird und die andere Druckkammern vom Gas entleert wird.

Vorteilhaft kann hierdurch erreicht werden, dass eine Blutpumpe erreicht wird, welche mechanisch autonom arbeitet und dabei nicht auf eine Elektronik oder äußere Vorgaben angewiesen ist und die Blutpumpe mit einem konstanten Gasstrom betrieben werden kann, wodurch keine externe Beschaltung notwendig ist.

Weiterhin kann vorteilhaft erreicht werden, dass die Blutpumpe ein hohes Maß an energetischer Effizienz erreicht.

Optional weist die Umschaltvorrichtung einen Magnet auf.

So ist unter anderem eine Anordnung von Magneten denkbar, in der ein Magnetpaar in der Mittellage die maximale Abstoßungskraft aufweist, wodurch eine bistabil ausgeführte Umschaltvorrichtung erreicht werden kann.

Vorteilhaft kann hierdurch eine bistabile und mechanisch autonome Blutpumpe mit einem hohen energetischen Wirkungsgrad erreicht werden.

Bevorzugt weist die Umschaltvorrichtung ein Hebelgetriebe auf.

Begrifflich sei hierzu Folgendes erläutert:
Ein "Hebelgetriebe" ist ein aus Stäben gebildetes Getriebe, wobei die Stäbe überwiegend eine kinematische Kette bilden.

Vorteilhaft kann hierdurch eine bistabile und mechanisch autonome Blutpumpe mit einem hohen energetischen Wirkungsgrad erreicht werden.

Optional weist die Umschaltvorrichtung eine Kulisse mit einer federbelasteten Rolle auf.

Begrifflich sei hierzu Folgendes erläutert:
Eine "Kulisse" ist ein Getriebeelement, welches einen Schlitz, einen Steg oder eine Nut aufweist. In oder an der Kulisse befindet sich ein beidseitig zwangsgeführter Kulissenstein, auf den die Bewegung der Kulisse übertragen wird.

Eine "federbelastete Rolle" ist eine Rolle, auf deren Bewegung die Kraft einer Feder einen Einfluss nimmt.

Vorteilhaft kann hierdurch eine bistabile und mechanisch autonome Blutpumpe mit einem hohen energetischen Wirkungsgrad erreicht werden.

Erfindungsgemäß weist ein Bereich zwischen den Membranen eine Koppelstange auf, insbesondere ist die Koppelstange mit den Wirkflächen verbunden, insbesondere ist die Koppelstange mit einer Membran und einer anderen Wirkfläche verbunden.

Begrifflich sei hierzu Folgendes erläutert:
Eine "Koppelstange" ist ein Bauteil, welches dazu eingerichtet ist die Bewegung von zwei Wirkflächen oder von einer Wirkfläche und einer Membran oder von zwei Membranen miteinander zu koppeln.

In einer besonders bevorzugten Ausführungsform überträgt sich die Endlage einer Membran auf das Steuerverfahren der Blutpumpe, insbesondere wirkt sich die Lage der Membran auf die Lage der Koppelstange aus und die Koppelstange schaltet beim Erreichen der Endlage einer Membran die Umschaltvorrichtung, sodass sich die Ventilstellungen in den jeweils anderen Schaltendzustand schalten und so beim Erreichen einer Membranendlage der Gasdruck in der Druckkammer reduziert wird und die andere Druckkammer mit Gasdruck beaufschlagt wird, sodass sich beide Membranen oder Wirkflächen nun in entgegengesetzter Richtung bewegen.

So kann bei einer geeigneten Ausführungsform die Membranendlage insbesondere erreicht werden, wenn eine Blutkammer nur noch etwa 10 % oder weniger ihres maximalen Volumens aufweist.

So kann sich der Kolben und damit die verbundenen Wirkflächen und/oder Membranen nach einem Arbeitsgang in die gegenüberliegende Richtung bewegen, indem die mit Druck beaufschlagte Druckkammer durch ein Umschalten durch die Umschaltvorrichtung gewechselt wird.

Weiterhin kann vorteilhaft erreicht werden, dass es nicht zu einer Beeinträchtigung im Materialverhalten der Blutkammer oder der Membran kommt.

Vorteilhaft kann hierdurch außerdem erreicht werden, dass die Blutpumpe mit einem konstanten Gasstrom betrieben werden kann, wodurch keine externe Beschaltung notwendig ist.

Optional weist die Koppelstange innen einen Hohlraum auf.

Vorteilhaft kann hierdurch erreicht werden, dass die Koppelstange unter gewissen Umständen im Inneren einen Gasstrom aufweisen kann. So kann mit einer hohlen Koppelstange ein Teil eines Bypasses erreicht werden.

Bevorzugt weist die Koppelstange ein erstes Bauteil und ein zweites Bauteil auf, wobei das erste Bauteil und das zweite Bauteil mit einer Feder verbunden sind.

Vorteilhaft kann hierdurch erreicht werden, dass die Bauteile der Koppelstange beim Erreichen einer durch die Feder definierten Kraft ihren Abstand zu einander verändern. Damit kann erreicht werden, dass sich die Wirkflächen der Druckkammern nicht synchron bewegen müssen.

So kann beim Überschreiten eines durch die Feder definierten Druckverhältnisses zwischen den Druckkammern eine ansonsten durch die Koppelstange gekoppelte Bewegung der Wirkflächen entkoppelt werden.

Optional weist die Koppelstange ein Ventil auf, welches dazu eingerichtet ist, dass ein designierter Gasstrom durch den Hohlraum in der Koppelstange und im weiteren Verlauf durch das Ventil in der Koppelstange und über einen Bypass aus einer Druckkammer ausströmen kann.

Begrifflich sei hierzu Folgendes erläutert:
Ein "Ventil" ist ein Bauteil zur Absperrung oder Regelung des Durchflusses von Fluiden. In Ventilen wird ein Verschlussteil bewegt, wodurch die Strömung reduziert oder unterbrochen wird.

Ein "Bypass" ist ein Strömungskanal, über welchen ein designierter Gasstrom strömen kann. Insbesondere ermöglicht der Bypass das Ausströmen von Gas aus einer Druckkammer, ohne dabei den Weg über das Gasauslassventil nehmen zu müssen. Der Bypass ist also ein alternativer Kanal zum Ausströmen von in einer Druckkammer befindlichem Gas.

In einer geeigneten Ausführungsform wird das Ventil passiv beim Erreichen eines Druckverhältnisses zwischen den beiden Druckkammern geöffnet, wodurch Gas aus der bedruckten Druckkammer über den Bypass ausströmen kann.

In einer vorteilhaften Ausführungsform sind die Bauteile der Koppelstange mit einer Feder verbunden. Übersteigt das Druckverhältnis in den Druckkammern einen durch die Vorspannung einer im Inneren der Koppelstange befindlichen Feder definierten Wert, öffnet sich das Ventil und Gas kann aus der bedruckten Druckkammer über den Bypass ausströmen.

Vorteilhaft kann hierdurch erreicht werden, dass eine weitere Schutzvorrichtung zur Ansauglimitierung erreicht werden kann.

Bevorzugt weist der Bypass eine Verschlusseinrichtung auf.

Begrifflich sei hierzu Folgendes erläutert:
Eine "Verschlusseinrichtung" weist ein mechanisches Verschlusselement auf, welches dazu eingerichtet einen Weg freizugeben, einen Weg zu schließen oder einen Strom auf dem Weg zu drosseln, insbesondere den Weg teilweise zu verschließen. Insbesondere ist eine Verschlusseinrichtung dazu eingerichtet einen Bypass zu öffnen, einen Bypass zu schließen oder einen Bypass zu drosseln, insbesondere teilweise zu verschließen.

Vorteilhaft kann hierdurch erreicht werden, dass das Ventil der Koppelstange durch Betätigen einer Verschlusseinrichtung versperrt werden kann. Damit kann erreicht werden, dass die Funktion des Ventils außer Kraft gesetzt wird.

Es sei ausdrücklich darauf hingewiesen, dass der Gegenstand des zweiten Aspekts mit dem Gegenstand des ersten Aspekts der Erfindung vorteilhaft kombinierbar ist.

Nach einem dritten Aspekt der Erfindung löst die Aufgabe eine Herz-Lungen-Maschine zum Fördern und Aufbereiten von Blut, wobei die Herz-Lungen-Maschine eine Blutzuführung und eine Blutabführung aufweist, wobei die Herz-Lungen-Maschine eine Blutpumpe nach einem der vorstehenden Ansprüche aufweist.

Es versteht sich, dass sich die Vorteile einer Blutpumpe zum Ansaugen und Verdrängen von Blut nach einem ersten oder zweiten Aspekt der Erfindung wie vorstehend beschrieben unmittelbar auf eine Herz-Lungen-Maschine zum Fördern und Aufbereiten von Blut erstrecken.

Bevorzugt weist die Herz-Lungen-Maschine einen Oxygenator auf.

Begrifflich sei hierzu Folgendes erläutert:
Ein "Oxygenator" ist ein medizintechnisches Produkt, das Blut mit Sauerstoff anreichert und Kohlenstoffdioxid aus dem Blut entfernt. Ein Oxgenator dient dem Gasaustausch im Blut.

Vorteilhaft kann hierdurch erreicht werden, dass die Funktion der Lunge ersetzt werden kann.

Optional weist die Herz-Lungen-Maschine einen Dialysator auf.

Begrifflich sei hierzu Folgendes erläutert:
Ein "Dialysator" ist ein medizintechnisches Produkt, welches dazu eingerichtet ist einen Stoffaustausch im Blut zu bewerkstelligen.

Vorteilhaft kann hierdurch erreicht werden, dass die Funktion der Niere ersetzt werden kann.

Bevorzugt weist die Herz-Lungen-Maschine einen Filter auf.

Begrifflich sei hierzu Folgendes erläutert:
Ein "Filter" ist ein medizintechnisches Produkt, welches dazu eingerichtet ist Partikel aus dem Blut zurückzuhalten.

Vorteilhaft kann hierdurch erreicht werden, dass das Blut von unerwünschten Partikeln gereinigt werden kann.

Optional weist die Herz-Lungen-Maschine eine Gasversorgung auf, insbesondere eine Sauerstoffversorgung.

Begrifflich sei hierzu Folgendes erläutert:
Eine "Gasversorgung" ist eine Baugruppe, die dazu eingerichtet ist einen Gasstrom zur Verfügung zu stellen.

Vorteilhaft kann hierdurch erreicht werden, dass einen Herz-Lungen-Maschine kontinuierlich mit Sauerstoff und Energie versorgt werden kann.

Nach einem vierten, nicht beanspruchten, Aspekt der Erfindung löst die Aufgabe ein Verfahren zum Betreiben einer extrakorporalen Blutpumpe nach einem ersten und/oder zweiten Aspekt der Erfindung.

Begrifflich sei hierzu Folgendes erläutert:
Eine "Blutzuführung" ist eine Verbindung zwischen dem Patienten und der Baugruppe Blutpumpe, welche dazu eingerichtet ist von Blut durchströmt zu werden.

Ein "Blutauslass" ist ein Bauteil durch welches ein designierter Blutstrom die Baugruppe Blutpumpe verlässt und welches dazu eingerichtet ist von Blut durchströmt zu werden.

In dem nicht beanspruchten Verfahren wird das Blut in einem ersten Verfahrensschritt durch Bewegen der Membran in der Blutpumpe und eine sich dadurch öffnende Rückströmdrossel im Bluteintrittskanal angesaugt, wobei das Blut durch den Bluteintrittskanal in die Blutkammer einströmt, sich in einem zweiten Verfahrensschritt die Rückströmdrossel am Bluteintrittskanal der Blutkammer schließt und das Blut in einem dritten Verfahrensschritt durch Bewegen der Membran aus der Blutkammer verdrängt wird, wobei die Rückströmdrossel am Blutein- trittskanal ein Rückströmen von Blut durch den Bluteintrittskanal verhindert oder abschwächt und das Blut durch den Blutaustrittskanal aus der Blutpumpe ausströmt, wobei die Bewegung der Koppelstange auf die Membran einwirkt und eine Bewegung der Membran beeinflusst.

Vorteilhaft kann hierdurch erreicht werden, dass die Blutpumpe besonders energieeffizient und blutschonend ist.

Außerdem kann vorteilhaft erreicht werden, dass alle von einem designierten Blutstrom durchströmten Bereiche besonderes gut ausgewaschen werden, sodass sich keine Thromben in der Blutpumpe bilden können.

Optional wird das Blut von zwei Blutkammern wechselseitig angesaugt und verdrängt, wobei wechselseitig eine Blutkammer Blut ansaugt und die andere Blutkammer Blut verdrängt, wobei die Rückströmdrossel im Verbindungsbereich der Blutaustrittskanäle ein Rückströmen von Blut in eine Blutkammer durch einen Blutaustrittskanal verhindert oder abschwächt.

Vorteilhaft kann hierdurch erreicht werden, dass eine pulsatiles Ansaugen und Verdrängen von Blut stattfindet, wobei die Blutpumpe mit einem konstanten Gasstrom betrieben werden kann, wodurch keine externe Beschaltung notwendig ist.

Vorteilhaft kann weiterhin hierdurch erreicht werden, dass die Blutpumpe besonders energieeffizient und blutschonend ist.

Außerdem kann vorteilhaft erreicht werden, dass alle von einem designierten Blutstrom durchströmten Bereiche besonderes gut ausgewaschen werden, sodass sich keine Thromben in der Blutpumpe bilden können.

Bevorzugt wirkt die Bewegung der Koppelstange auf die Membran ein und beeinflusst eine Bewegung der Membran.

Vorteilhaft kann hierdurch erreicht werden, dass eine Druckkammer stets nur mit einem Überdruck jedoch nie mit einem Unterdruck versorgt werden muss. Die jeweils mit Überdruck bedruckte Druckkammer bestimmt die Bewegung und schleppt die Membran oder Wirkfläche der gegenüberliegenden Druckkammer mit, sodass hier kein Unterdruck zum Auslassen des Gases aufgebracht werden muss.

Diese Kopplung kann vorteilhaft ermöglichen, dass die Blutpumpe mit einem konstanten Gasstrom betrieben werden kann, wodurch keine externe Beschaltung notwendig ist.

Optional wird die Antriebseinheit mit Gas betrieben, wobei das Gas durch einen Gaseinlass in die Antriebseinheit einströmt, wobei das Gas durch einen Gasauslass aus der Antriebseinheit ausströmt, wobei ein Differenzdruck zwischen der Blutkammer und der Druckkammer auf die Membran einwirkt und eine Bewegung der Membran beeinflusst.

Vorteilhaft kann hierdurch erreicht werden, dass die Energiezufuhr der Blutpumpe durch einen Gasstrom bereitgestellt werden kann, wodurch eine besonders einfache und robuste Blutpumpe möglich ist.

Bevorzugt strömt das Gas durch einen Gaseinlass in die Antriebseinheit ein und im weiteren Verlauf durch ein geöffnetes Gaseinlassventil in eine Druckkammer ein, wobei das Gasauslassventil dieser Druckkammer verschlossen ist, sodass sich der Druck in der Druckkammer erhöht und die Bewegung der Membran beeinflusst, wobei sich die Membran direkt oder zeitlich verzögert in Richtung der Blutkammer bewegt.

Vorteilhaft kann hierdurch erreicht werden, dass die eine Membran und damit auch den Blutstrom in der angrenzenden Blutkammer verdrängende Druckkammer mit Gasdruck betrieben werden kann, also seine Energie aus dem Druck des zugeführten Gases beziehen kann.

Optional strömt das Gas durch ein Gasauslassventil aus einer Druckkammer aus und im weiteren Verlauf durch einen Gasauslass aus der Antriebseinheit aus, wobei das Gaseinlassventil dieser Druckkammer verschlossen ist, sodass sich der Druck in der Druckkammer reduziert und die Bewegung der Membran beeinflusst, wobei sich die Membran direkt oder zeitlich verzögert in Richtung der Druckkammer bewegt.

Vorteilhaft kann hierdurch erreicht werden, dass das zum Betreiben der Antriebseinheit verwendete Gas die Blutpumpe wieder verlassen kann.

Bevorzugt strömt das Gas wechselseitig in eine erste Druckkammer ein, während Gas aus einer zweiten Druckkammer ausströmt, und die Umschaltvorrichtung bei Erreichen einer Membranendlage oder einer Wirkflächenendlage das Gaseinlassventil und das Gasauslassventil umschaltet, sodass im weiteren Verlauf Gas in die zweite Druckkammer einströmt, während Gas aus der ersten Druckkammer ausströmt.

Vorteilhaft kann hierdurch erreicht werden, dass eine Blutpumpe mit einem pulsatilen Ansaugen und Verdrängen von Blut möglich ist, wobei die Blutpumpe mit einem konstanten Gasstrom betrieben werden kann, wodurch keine externe Beschaltung notwendig ist.

Optional wird die Umschaltvorrichtung in bistabilen Schaltzuständen geschaltet, sodass nach einem Schaltvorgang nur eine Druckkammer eine Gasverbindung mit dem Gaseinlass und die andere Druckkammer eine Gasverbindung mit dem Gasauslass aufweist.

Vorteilhaft kann hierdurch erreicht werden, dass die Blutpumpe nicht zwischen den designierten Schaltzuständen hängen bleiben und kann, wodurch die Funktion der Blutpumpe beeinträchtigt wäre oder sogar ausfallen würde. Die Blutpumpe arbeitet mechanisch autonom mit bistabilen Schaltzustände und ist nicht auf eine externe Steuerung angewiesen, wodurch ein besonders sicheres und robustes Verhalten einer Blutpumpe vorteilhaft erreicht werden kann.

Bevorzugt strömt das Gas bei einem betragsmäßigen Überschreiten eines Differenzdruckes zwischen den Druckkammern über das Ventil aus einer Druckkammer aus.

Vorteilhaft kann hierdurch erreicht werden, dass eine zusätzliche Schutzvorrichtung zur Ansauglimitierung ermöglicht wird. Somit können Risiken beim Einsatz einer Blutpumpe reduziert werden.

Optional verschließt die Verschlusseinrichtung den Bypass, sodass kein Gas oder nur ein reduzierter Gasstrom durch das Ventil strömen kann.

Vorteilhaft kann hierdurch erreicht werden, dass die mit dem Bypass und dem zugehörigen Ventil ermöglichte Schutzfunktion zu Gunsten einer höheren Pumpenleistung deaktiviert werden kann.

Nach einem fünften, nicht beanspruchten, Aspekt der Erfindung löst die Aufgabe ein Verfahren zum Betreiben einer Herz-Lungen-Maschine nach dem dritten Aspekt der Erfindung.

Hierbei wird die Blutpumpe der Herz-Lungen-Maschine mit einem Verfahren nach dem vierten Aspekt der Erfindung betrieben.

Vorteilhaft kann hierdurch erreicht werden, dass sich die Vorteile eines Verfahrens zum Betreiben einer Blutpumpe nach einem vierten Aspekt der Erfindung wie vorstehend beschrieben unmittelbar auf ein Verfahren zum Betreiben einer Herz-Lungen-Maschine erstrecken.

Optional wird das Blut der Blutpumpe und im weiteren Verlauf dem Oxygenator und/oder dem Filter und/oder dem Dialysator zugeführt.

Vorteilhaft kann hierdurch erreicht werden, dass das dem Patienten entnommene Blut mit Sauerstoff angereichert werden kann, von Kohlendioxid und ungewünschten Partikeln befreit werden kann und ein Stoffaustausch im Blut stattfinden kann. Somit können die Funktionen des Herzens, der Lunge und der Nieren teilweise oder vollständig ersetzt werden.

Nachstehend wird die Erfindung anhand von Ausführungsbeispielen unter Bezugnahme auf die Zeichnung näher erläutert. Dort zeigen
- Fig. 1: schematisch eine Herz-Lungen-Maschine,
- Fig. 2: schematisch eine Blutpumpe,
- Fig. 3: schematisch eine Umschaltvorrichtung,
- Fig. 4: schematisch eine Ausführung einer Koppelstangenanordnung mit Ventil und Bypass,
- Fig. 5: schematisch eine Blutpumpe mit Bypass und Verschlusseinrichtung,
- Fig. 6: schematisch einen Verbindungsbereich der Blutaustrittskanäle und
- Fig. 7: schematisch eine Blutkammereinheit mit Bluteintrittskanal, Blutaustrittskanal und Rückströmdrossel.

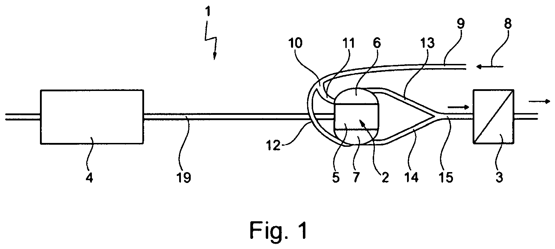

Die Herz-Lungen-Maschine 1 in Figur 1 besteht im Wesentlichen aus einer Blutpumpe 2, einem Oxygenator 3 und einer Versorgungseinheit 4.

Die Blutpumpe 2 besteht im Wesentlichen aus einer Antriebseinheit 5 und zwei Blutkammern 6, 7.

Ein designierter Blutstrom (nicht dargestellt) strömt in einer designierten Blutstromeintrittsrichtung 8 in einer Blutzuführung 9 vom Patienten (nicht dargestellt) in Richtung der Blutpumpe 2.

Bevor der designierte Blutstrom (nicht dargestellt) die Blutpumpe 2 erreicht, teilt er sich in einem Strömungsteiler 10 auf und strömt stromab des Strömungsteilers 10 in zwei Bluteintrittskanälen 11, 12 in je eine Blutkammer 6, 7 wird dort durch das Einwirken der Antriebseinheit 5 bedruckt und verlässt die Blutpumpe 2 über je einen Blutaustrittskanal 13, 14.

Am Ende der Blutaustrittskanäle 13, 14 strömt der designierte Blutstrom (nicht dargestellt) in einem Verbindungsbereich 14 zusammen und von dort stromab weiter in Richtung des Oxygenators 3.

Der designierte Blutstrom tritt in der Blutströmungsrichtung 16 in den Oxygenator ein und in Blutströmungsrichtung 17 aus dem Oxygenator 3 aus.

Stromab des Oxygenators 3 strömt der designierte Blutstrom (nicht dargestellt) durch eine Blutabführung 18 zurück zum Patienten (nicht dargestellt).

Die Versorgungseinheit 4 versorgt die Blutpumpe 2, insbesondere die Antriebseinheit 5 der Blutpumpe 2, mit einem Gasstrom (nicht dargestellt) über eine Gaszuführung 19 und liefert der Blutpumpe 2 damit die für das Pumpen des designierten Blutstromes (nicht dargestellt) benötigte Energie.

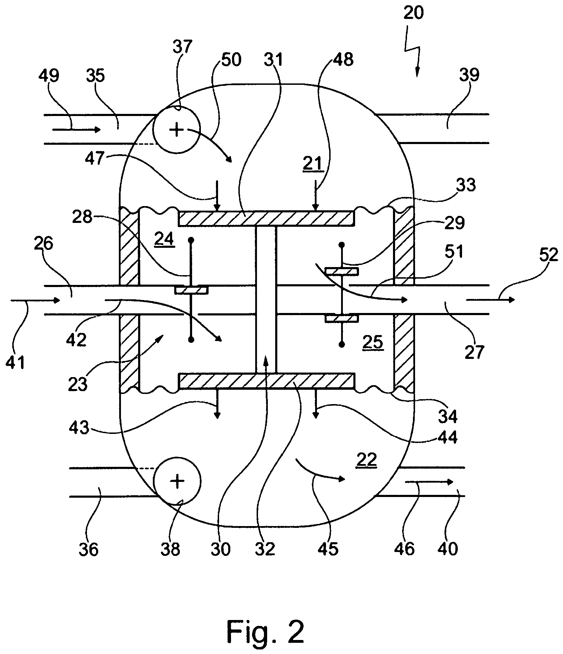

Die Blutpumpe 20 in Figur 2 besteht im Wesentlichen aus zwei Blutkammern 21, 22 und einer Antriebseinheit 23.

Die Antriebseinheit 23 besteht im Wesentlichen aus zwei Druckkammern 24, 25, einem Gaseinlass 26, einem Gasauslass 27, einem Gaseinlassventil 28, einem Gasauslassventil 29 und einer Koppelstange 30.

Die Koppelstange 30 weist an ihren Enden je eine Wirkfläche 31, 32 auf.

Die Blutkammer 21 ist von der Druckkammer 24 durch die Wirkfläche 31 und die Membran 33 getrennt, sodass ein Fluid (nicht dargestellt) weder von der Blutkammer 21 in die Druckkammer 24 noch von der Druckkammer 24 in die Blutkammer 21 strömen kann.

Die Blutkammer 22 ist von der Druckkammer 25 durch die Wirkfläche 32 und die Membran 34 getrennt, sodass ein Fluid (nicht dargestellt) weder von der Blutkammer 22 in die Druckkammer 25 noch von der Druckkammer 25 in die Blutkammer 22 strömen kann.

Das Gaseinlassventil 28 kann so geschaltet werden, dass ein designierter Gasstrom (nicht dargestellt) über den Gaseinlass 26 in die Druckkammer 24 oder die Druckkammer 25 strömen kann.

Das Gaseinlassventil 29 kann so geschaltet werden, dass ein designierter Gasstrom (nicht dargestellt) aus der Druckkammer 24 oder der Druckkammer 25 in den Gasauslass 27 strömen kann.

Ein designierter Blutstrom (nicht dargestellt) strömt durch einen Bluteintrittskanal 35 und eine Rückströmdrossel 37 in die Blutkammer 21 ein und vor dort stromab über den Blutaustrittskanal 39 aus der Blutkammer 21 wieder aus.

Ein designierter Blutstrom (nicht dargestellt) strömt durch einen Bluteintrittskanal 36 und eine Rückströmdrossel 38 in die Blutkammer 22 ein und vor dort stromab über den Blutaustrittskanal 40 aus der Blutkammer 22 wieder aus.

Die Blutpumpe 20 in Figur 2 kann beispielhaft folgenden Zustand aufweisen. Ein designierter Gasstrom (nicht dargestellt) strömt in einer Gaseinströmrichtung 41 durch den Gaseinlass 26 in die Antriebseinheit 23 ein. Dort strömt der designierte Gasstrom (nicht dargestellt) über das Gaseinlassventil 28 in der Gasströmungsrichtung 42 in die Drucckammer 25 ein. Da das Gasauslassventil 29 die Druckkammer 25 verschließt, erhöht sich der Gasdruck in der Druckkammer 25.

Der Gasdruck in der Druckkammer 25 wirkt auf die Wirkfläche 32 und die Membran 34 ein und wird so hoch, dass sich die Wirkfläche 32 und die Membran 34 mit der Bewegungsrichtung 43, 44 in Richtung der Blutkammer 22 verschieben.

Dadurch reduziert sich das Volumen der Blutkammer 22, die Rückströmdrossel 38 verschließt sich und ein designierter Blutstrom (nicht dargestellt) strömt in einer Blutströmungsrichtung 45 auf den Blutaustrittskanal 40 zu und weiter durch den Blutaustrittskanal 40 in Blutströmungsrichtung 46 aus der Blutpumpe 20 heraus.

Die Bewegungsrichtung 43, 44 der Wirkfläche 32 wird über die Koppelstange 30 auf die Bewegungsrichtung 47, 48 der Wirkfläche 31 übertragen, so dass sich die Wirkfläche 31 und die Membran 33 derart bewegen, dass das Volumen der Blutkammer 21 steigt.

Dadurch wird ein designierter Blutstrom (nicht dargestellt) durch den Bluteintrittskanal 35 in der Bluteinströmrichtung 49 und weiter stromab durch die sich dadurch öffnende Rückströmdrossel 37 in Blutströmungsrichtung 50 in die Blutkammer 21 angesaugt.

Gleichzeitig führt die Bewegungsrichtung 47, 48 der Wirkfläche 31 und der Membran 33 zu einer Reduktion des Volumens der Druckkammer 24, wodurch ein designierter Gasstrom (nicht dargestellt) in der Gasströmungsrichtung 51 durch das geöffnete Gasauslassventil 29 aus der Druckkammer 24 in den Gasauslass 27 strömt und von dort weiter stromab in der Gasausströmrichtung 52 die Antriebseinheit 23 verlässt.

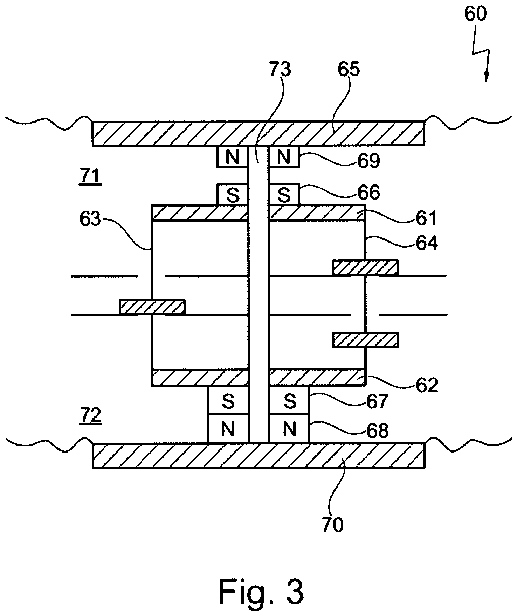

Die Umschaltvorrichtung 60 in Figur 3 besteht im Wesentlichen aus einem oberen Schaltteil 61, einem unteren Schaltteil 62 sowie aus den Magneten 66, 67, 68, 69.

Das obere Schaltteil 61 und das untere Schaltteil 62 verbinden jeweils das Gaseinlassventil 63 und das Gasauslassventil 64 an der oberen und der unteren Seite des Gaseinlassventils 63 und des Gasauslassventils 64.

Die Stellung des Gaseinlassventils 63 und des Gasauslassventils 64 entscheidet darüber in welche der Druckkammern 71, 72 ein designierter Gasstrom (nicht dargestellt) einströmt oder ausströmt und wird über die Umschaltvorrichtung 60 umgeschaltet.

In der Druckkammer 71 befinden sich die Magnete 66, 69 mit voneinander abweichender Polarisierung. Der Magnet 66 ist mit dem oberen Schaltteil 61 verbunden. Der Magnet 69 ist mit der Wirkfläche 65 verbunden und bewegt sich folglich mit der Wirkfläche 65 mit. Da die Wirkfläche 65 über die Koppelstange 73 mit der Wirkfläche 70 verbunden ist, bewegen sich auch die Koppelstange 73 und die Wirkfläche 70 gleichermaßen mit.

In der Druckkammer 72 befinden sich die Magnete 67, 68 mit voneinander abweichender Polarisierung. Der Magnet 67 ist mit dem unteren Schaltteil 62 verbunden. Der Magnet 68 ist mit der Wirkfläche 70 verbunden und bewegt sich folglich mit der Wirkfläche 70 mit. Da die Wirkfläche 70 über die Koppelstange 73 mit der Wirkfläche 65 verbunden ist, bewegen sich auch die Koppelstange 73 und die Wirkfläche 65 gleichermaßen mit.

Die Magnete 66, 69 sowie die Magnete 67, 68 weisen jeweils eine abweichende Polarisierung auf und ziehen sich folglich an. Die Anziehungskraft der Magnete 66, 69 sowie der Magnete 67, 68 ist dabei von dem Abstand zwischen den jeweiligen Magneten abhängig. Berühren sich die Magnete 66, 69 oder die Magnete 67, 68 ist deren jeweilige Anziehungskraft am größten.

Durch die Anordnung der Magnete 66, 67, 68, 69 in Kombination mit der Gestaltung der Umschaltvorrichtung 60 und der von den Magneten 66, 67, 68, 69 ausgehenden Anziehungskraft wird bei der in der Beschreibung zu Figur 2 bereits beschriebenen Kinematik der Blutpumpe 20 mit Umschaltvorrichtung 60 eine bistabile Ausführung der Umschaltvorrichtung 60 erreicht, sodass bistabile Schaltzustände ermöglicht werden.

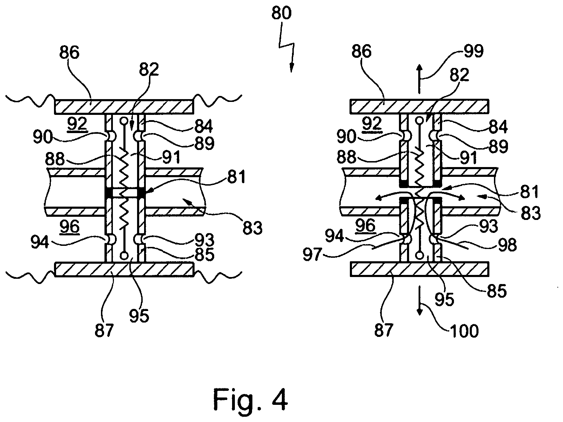

Die Ausführungsform einer Koppelstangenanordnung 80 mit Ventil und Bypass in Figur 4 (linke Teilfigur zeigt die Ausführungsform der Koppelstangenanordnung 80 mit geschlossenem Ventil 81, rechte Teilfigur zeigt die Ausführungsform der Koppelstangenanordnung 80 mit geöffnetem Ventil 81) besteht im Wesentlichen aus einem Ventil 81, einer Koppelstange 82 und einem Bypass 83.

Die Koppelstange 82 weist ein oberes Koppelstangenbauteil 84 und ein unteres Koppelstangenbauteil 85 auf. Das obere Koppelstangenbauteil 84 ist fest mit der Wirkfläche 86 verbunden. Das untere Koppelstangenbauteil 85 ist fest mit der Wirkfläche 87 verbunden.

Das obere Koppelstangenbauteil 84 und das untere Koppelstangenbauteil 85 werden durch eine Feder 88 am Ort des Ventils 81 aufeinandergepresst. Wirkt eine Kraft auf die Wirkflächen 86, 87 ein, welche größer ist als die Federkraft und in entgegengesetzter Richtung zur Federkraft wirkt, so öffnet sich das Ventil 81 (rechte Teilfigur).

Das obere Koppelstangenbauteil 84 ist innen hohl und weist die Bohrungen 89, 90 auf, welche den Hohlraum 91 des oberen Koppelstangenbauteils 84 mit der Druckkammer 92 verbinden können. Die Anordnung der Bohrungen 89, 90 ist derart gestaltet, dass sie den Hohlraum 91 des oberen Koppelstangenbauteils 84 mit der Druckkammer 92 nur freigeben, wenn diese mehr als 50 % ihres nominalen Druckkammervolumens aufweist.

Das untere Koppelstangenbauteil 85 ist innen hohl und weist die Bohrungen 93, 94 auf, welche den Hohlraum 95 des unteren Koppelstangenbauteils 85 mit der Druckkammer 96 verbinden können. Die Anordnung der Bohrungen 93, 94 ist derart gestaltet, dass sie den Hohlraum 95 des unteren Koppelstangenbauteils 85 mit der Druckkammer 96 nur freigeben, wenn diese mehr als 50 % ihres nominalen Druckkammervolumens aufweist.

Insbesondere ist die Koppelstange 82 so gestaltet, dass in keinem Zustand gleichzeitig die Bohrungen 89, 90 den Hohlraum 91 des oberen Koppelstangenbauteils 84 mit der Druckkammer 92 verbinden und die Bohrungen 93, 94 den Hohlraum 95 des unteren Koppelstangenbauteils 85 mit der Druckkammer 96 verbinden. So wird sichergestellt, dass die Hohlräume 91, 95 der Koppelstange 82 nicht den Druck zwischen den Drucckammern 92, 96 ausgleichen. Insbesondere wird ein solcher Druckausgleich ausgeschlossen.

Wirkt eine Kraft 99, 100 auf die Wirkflächen 86, 87 ein, welche größer ist als die Federkraft und in entgegengesetzter Richtung zur Federkraft wirkt, so öffnet sich das Ventil 81 (rechte Teilfigur). Weist die Druckkammer 96 mehr als 50 % ihres nominalen Druckkammervolumens auf, so strömt ein designierter Gasstrom (nicht dargestellt) aus der Drucckammer 96 in Gasströmungsrichtung 97, 98 durch die Bohrung 93, 94 in den Hohlraum 95, durch das geöffnete Ventil 81 und weiter stromab in den Bypass 83.

Alternativ (nicht dargestellt) strömt ein designierter Gasstrom (nicht dargestellt) aus der Druckkammer 92 in Gasströmungsrichtung (nicht dargestellt) durch die Bohrung 89, 90 in den Hohlraum 91, durch das geöffnete Ventil 81 und weiter stromab in den Bypass 83, sofern die Kraft 99, 100 auf die Wirkflächen 86, 87 größer ist als die Federkraft, in entgegengesetzter Richtung zur Federkraft wirkt und die Druckkammer 92 mehr als 50 % ihres nominalen Druckkammervolumens aufweist.

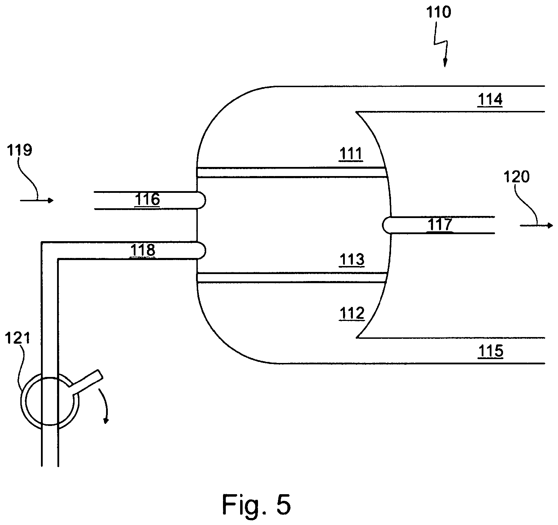

Die Blutpumpe 110 in Figur 5 besteht im Wesentlichen aus den Blutkammern 111, 112 und einer Antriebseinheit 113.

Die Blutkammern 111, 112 weisen jeweils einen Bluteintrittskanal (nicht dargestellt) und einen Blutaustrittskanal 114, 115 auf.

Die Antriebseinheit 113 weist einen Gaseinlass 116, einen Gasauslass 117 und einen Bypass 118 auf., sodass ein designierter Gasstrom (nicht dargestellt) in Gaseinströmrichtung 119 durch den Gaseinlass 116 in die Antriebseinheit 113 einströmen und durch den Gasauslass 117 in Gasausströmrichtung 120 ausströmen kann.

Öffnet sich das Ventil 81 im inneren der Antriebseinheit 113, kann ein designierter Gasstrom (nicht dargestellt) durch den Bypass 118 aus der Antriebseinheit 113 ausströmen.

Der Bypass 118 weist eine Verschlusseinrichtung 121 auf, welche es ermöglicht den Bypass 118 zu öffnen, sodass ein designierter Gasstrom (nicht dargestellt) bei Öffnung des Ventils 81 durch den Bypass strömen kann, zu schließen, sodass ein designierter Gasstrom (nicht dargestellt) bei Öffnung des Ventils 81 nicht durch den Bypass strömen kann, oder zu drosseln, sodass ein designierter Gasstrom (nicht dargestellt) bei Öffnung des Ventils 81 nur gedrosselt durch den Bypass strömen kann.

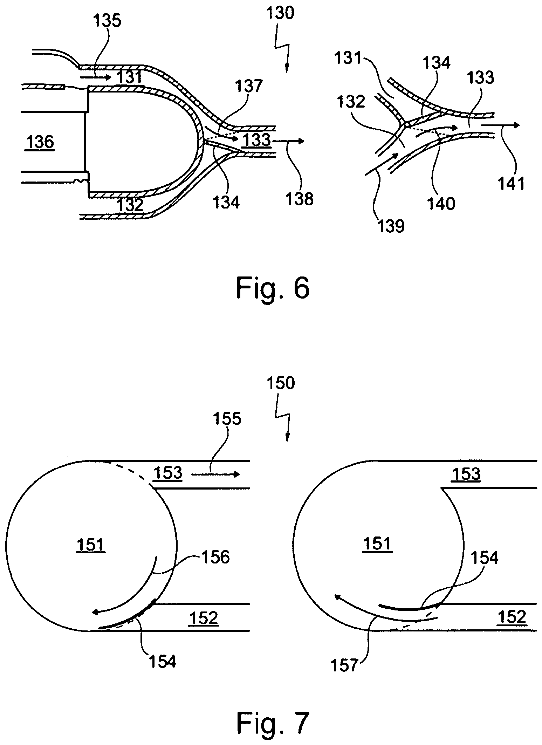

Der Verbindungsbereich 130 in Figur 6 (linke Teilfigur und rechte Teilfigur zeigen alternative Blutströmwege) besteht im Wesentlichen aus den Blutaustrittskanälen 131, 132, welche sich im Verbindungsbereich 130 treffen, der Blutabführung 133 zum Pateinten (nicht dargestellt) und einer Rückströmdrossel 134.

Ein designierter Blutstrom (nicht dargestellt) kann in Blutströmungsrichtung 135 aus der Blutpumpe 136 durch den Blutaustrittskanal 131 ausströmen, trifft dann weiter stromab auf den Verbindungsbereich 130, welcher in Blutströmungsrichtung 137 durchströmt wird, und die Rückströmdrossel 134, welche in Blutströmungsrichtung 137 passiert wird. Weiter stromab strömt der designierte Blutstrom (nicht dargestellt) aus dem Verbindungsbereich 130 in Blutströmungsrichtung 138 durch die Blutabführung 133 zurück zum Patienten (nicht dargestellt).

Ein designierter Blutstrom (nicht dargestellt) kann in Blutströmungsrichtung 139 aus der Blutpumpe 136 durch den Blutaustrittskanal 132 ausströmen, trifft dann weiter stromab auf den Verbindungsbereich 130, welcher in Blutströmungsrichtung 140 durchströmt wird, und die Rückströmdrossel 134, welche in Blutströmungsrichtung 140 passiert wird. Weiter stromab strömt der designierte Blutstrom (nicht dargestellt) aus dem Verbindungsbereich 130 in Blutströmungsrichtung 141 durch die Blutabführung 133 zurück zum Patienten (nicht dargestellt).

Die Rückströmdrossel 134 verhindert oder dämpft ein etwaiges Rückströmen eines designierten Blutstroms (nicht dargestellt) durch den jeweils nicht mit der Blut verdrängenden Blutkammer (nicht dargestellt) der Blutpumpe 136 verbundenen Blutaustrittskanal 131, 132.

Eine Blutkammereinheit 150 in Figur 7 (linke Teilfigur zeigt eine Verdrängen von Blut aus der Blutkammer, rechte Teilfigur zeigt ein Ansaugen von Blut in die Blutkammer) besteht im Wesentlichen aus einem Blutkammer 151, einem Bluteintrittskanal 152, einem Blutaustrittskanal 153 und einer Rückströmdrossel 154.

Beim Verdrängen eines designierten Blutstroms (nicht dargestellt) strömt der designierte Blutstrom (nicht dargestellt) durch den Blutaustrittskanal 153 in Blutströmungsrichtung 155 aus der Blutkammer 151 aus. Dabei kommt es zu einer Zirkulationsbewegung 156 des designierten Blutstroms (nicht dargestellt) in der Blutkammer 151.

Beim Verdrängen eines designierten Blutstroms (nicht dargestellt) aus der Blutkammer 153 verhindert oder drosselt die Rückströmdrossel 154 ein Ausströmen eines designierten Blutstroms (nicht dargestellt) aus der Blutkammer 151 durch den Bluteintrittskanal 152.

Beim Ansaugen eines designierten Blutstroms (nicht dargestellt) strömt der designierte Blutstrom (nicht dargestellt) durch den Bluteintrittskanal 152 in Blutströmungsrichtung 157 und durch die Rückströmdrossel 154 in die Blutkammer 151 ein.

### Liste der verwendeten Bezugszeichen

- 1: Herz-Lungen-Maschine
- 2: Blutpumpe
- 3: Oxygenator
- 4: Versorgungseinheit
- 5: Antriebseinheit
- 6: Blutkammer
- 7: Blutkammer
- 8: Blutstromeintrittsrichtung
- 9: Blutzuführung
- 10: Strömungsteiler
- 11: Bluteintrittskanal
- 12: Bluteintrittskanal
- 13: Blutaustrittskanal
- 14: Blutaustrittskanal
- 15: Verbindungsbereich
- 16: Blutströmungsrichtung
- 17: Blutströmungsrichtung
- 18: Blutabführung
- 19: Gaszuführung
- 20: Blutpumpe
- 21: Blutkammer
- 22: Blutkammer
- 23: Antriebseinheit
- 24: Druckkammer
- 25: Druckkammer
- 26: Gaseinlass
- 27: Gasauslass
- 28: Gaseinlassventil
- 29: Gasauslassventil
- 30: Koppelstange
- 31: Wirkfläche
- 32: Wirkfläche
- 33: Membran
- 34: Membran
- 35: Bluteintrittskanal
- 36: Bluteintrittskanal
- 37: Rückströmdrossel
- 38: Rückströmdrossel
- 39: Blutaustrittskanal
- 40: Blutaustrittskanal
- 41: Gaseinströmrichtung
- 42: Gasströmungsrichtung
- 43: Bewegungsrichtung
- 44: Bewegungsrichtung
- 45: Blutströmungsrichtung
- 46: Blutströmungsrichtung
- 47: Bewegungsrichtung
- 48: Bewegungsrichtung
- 49: Bluteinströmrichtung
- 50: Blutströmungsrichtung
- 51: Gasströmungsrichtung
- 52: Gasausströmrichtung
- 60: Umschaltvorrichtung
- 61: oberes Schaltteil
- 62: unteres Schaltteil
- 63: Gaseinlassventil
- 64: Gasauslassventil
- 65: Wirkfläche
- 66: Magnet
- 67: Magnet
- 68: Magnet
- 69: Magnet
- 70: Wirkfläche
- 71: Druckkammer
- 72: Druckkammer
- 73: Koppelstange
- 80: Koppelstangenanordnung
- 81: Ventil
- 82: Koppelstange
- 83: Bypass
- 84: oberes Koppelstangenbauteil
- 85: unteres Koppelstangenbauteil
- 86: Wirkfläche
- 87: Wirkfläche
- 88: Feder
- 89: Bohrung
- 90: Bohrung
- 91: Hohlraum
- 92: Druckkammer
- 93: Bohrung
- 94: Bohrung
- 95: Hohlraum
- 96: Druckkammer
- 97: Gasströmungsrichtung
- 98: Gasströmungsrichtung
- 99: Kraft
- 100: Kraft
- 110: Blutpumpe
- 111: Blutkammer
- 112: Blutkammer
- 113: Antriebseinheit
- 114: Blutaustrittskanal
- 115: Blutaustrittskanal
- 116: Gaseinlass
- 117: Gasauslass
- 118: Bypass
- 119: Gaseinströmrichtung
- 120: Gasausströmrichtung
- 121: Verschlusseinrichtung
- 130: Verbindungsbereich
- 131: Blutaustrittskanal
- 132: Blutaustrittskanal
- 133: Blutabführung
- 134: Rüclströmdrossel
- 135: Blutströmungsrichtung
- 136: Blutpumpe
- 137: Blutströmungsrichtung
- 138: Blutströmungsrichtung
- 139: Blutströmungsrichtung
- 140: Blutströmungsrichtung
- 141: Blutströmungsrichtung
- 150: Blutkammereinheit
- 151: Blutkammer
- 152: Bluteintrittskanal
- 153: Blutaustrittskanal
- 154: Rückströmdrossel
- 155: Blutströmungsrichtung
- 156: Zirkulationsbewegung
- 157: Blutströmungsrichtung

## Patentansprüche

1. Extrakorporale Blutpumpe zum Ansaugen und Verdrängen von Blut, wobei die Blutpumpe ein oder zwei Blutkammern und eine mechanische Antriebseinheit aufweist, wobei jede der Blutkammern eine Membran, einen Bluteintrittskanal und einen Blutaustrittskanal aufweist,
- wobei im Falle der Blutpumpe mit der einen Blutkammer die Antriebseinheit an die Blutkammer angrenzt, wobei die Antriebseinheit einem Gaseinlass und einem Gasauslass sowie zwei Druckkammern aufweist, wobei jede Druckkammer eine Wirkfläche aufweist, wobei eine Blutkammer über eine Membran von einer Druckkammer getrennt ist, wobei die Antriebseinheit in Wirkverbindung zur Lage der Membran steht, und wobei ein Bereich zwischen der Wirkfläche und der Membran eine Koppelstange aufweist, welche dazu eingerichtet ist, die Bewegung von der Wirkfläche und der Membran miteinander zu koppeln;
- wobei im Falle der Blutpumpe mit zwei Blutkammern die Antriebseinheit zwischen den Blutkammern angeordnet ist, die Blutaustrittskanäle beider Blutkammern miteinander verbunden sind und ein Bereich zwischen den Membranen eine Koppelstange aufweist, welche dazu eingerichtet ist, die Bewegung der zwei Membranen miteinander zu koppeln, und dass die Antriebseinheit einen Gaseinlass und einen Gasauslass sowie eine Druckkammer aufweist, wobei die Druckkammer über eine Membran von einer Blutkammer getrennt ist, wobei die Antriebseinheit in Wirkverbindung zur Lage der Membran steht.

2. Extrakorporale Blutpumpe nach Anspruch 1, **dadurch gekennzeichnet, dass** die Bluteintrittskanäle beider Blutkammern miteinander verbunden sind,

3. Extrakorporale Blutpumpe nach einem der Ansprüche 1 oder 2, **dadurch gekennzeichnet, dass** ein Verbindungsbereich der Blutaustrittskanäle eine Rückstromdrossel aufweist,

4. Extrakorporale Blutpumpe nach einem der vorstehenden Ansprüche, **dadurch gekennzeichnet, dass** die Antriebseinheit, zwei Druckkammern aufweist, wobei jede Druckkammer über eine Membran an eine Blutkammer angrenzt.

5. Extrakorporale Blutpumpe nach einem der vorstehenden Ansprüche, **dadurch gekennzeichnet, dass** eine Blutkammer einen rotationssymmetrischen Bereich aufweist.

6. Extrakorporale Blutpumpe nach einem der vorstehenden Ansprüche, **dadurch gekennzeichnet, dass** ein Bluteintrittskanal und ein Blutaustrittskanal vorwiegend in Umfangsrichtung an den rotationssymmetrischen Bereich einer Blutkammer angeordnet sind.

7. Extrakorporale Blutpumpe nach einem der vorstehenden Ansprüche, **dadurch gekennzeichnet, dass** ein Bluteintrittskanal eine Rückströmdrossel aufweist.

8. Extrakorporale Blutpumpe nach einem der vorstehenden Ansprüche, **dadurch gekennzeichnet, dass** die Antriebseinheit ein Gaseinlassventil und ein Gasauslassventil sowie eine Umschaltvorrichtung aufweist, wobei das Gaseinlassventil und das Gasauslassventil gegenüber einem Gasströmweg eine Verschlussstellung und eine Öffnungsstellung aufweisen, wobei die Umschaltvorrichtung zwei Schaltendzustände aufweist, insbesondere weist die Umschaltvorrichtung genau zwei Schaltzustände auf.

9. Extrakorporale Blutpumpe nach Anspruch 8, **dadurch gekennzeichnet, dass** ein Schaltendzustand für eine Blutkammer das Gaseinlassventil in Öffnungsstellung und das Gasauslassventil in Verschlussstellung oder das Gaseinlassventil in Verschlussstellung und das Gasauslassventil in Öffnungsstellung aufweist.

10. Extrakorporale Blutpumpe nach einem der Ansprüche 8 oder 9, **dadurch gekennzeichnet, dass** die Umschaltvorrichtung einen Magnet aufweist.

11. Extrakorporale Blutpumpe nach einem der Ansprüche 8 bis 10, **dadurch gekennzeichnet, dass** die Umschaltvorrichtung ein Hebelgetriebe aufweist.

12. Extrakorporale Blutpumpe nach einem der Ansprüche 8 bis 11, **dadurch gekennzeichnet, dass** die Umschaltvorrichtung eine Kulisse mit einer federbelasteten Rolle aufweist.

13. Extrakorporale Blutpumpe nach einem der vorstehenden Ansprüche, **dadurch gekennzeichnet, dass** die Koppelstange innen einen Hohlraum aufweist.

14. Extrakorporale Blutpumpe nach einem der vorstehenden Ansprüche, **dadurch gekennzeichnet, dass** die Koppelstange ein erstes Bauteil und ein zweites Bauteil aufweist, wobei das erste Bauteil und das zweite Bauteil mit einer Feder verbunden sind.

15. Extrakorporale Blutpumpe nach einem der vorstehenden Ansprüche, **dadurch gekennzeichnet, dass** die Koppelstange ein Ventil aufweist, welches dazu eingerichtet ist, dass ein designierter Gasstrom durch den Hohlraum in der Koppelstange und im weiteren Verlauf durch das Ventil in der Koppelstange und über einen Bypass aus einer Druckkammer ausströmen kann.

16. Extrakorporale Blutpumpe nach Anspruch 15, **dadurch gekennzeichnet, dass** der Bypass eine Verschlusseinrichtung aufweist.

17. Herz-Lungen-Maschine zum Fördern und Aufbereiten von Blut, wobei die Herz-Lungen-Maschine eine Blutzuführung und eine Blutabführung aufweist, **dadurch gekennzeichnet, dass** die Herz-Lungen-Maschine eine Blutpumpe nach einem der vorstehenden Ansprüche aufweist.

18. Herz-Lungen-Maschine nach Anspruch 17, **dadurch gekennzeichnet, dass** die Herz-Lungen-Maschine einen Oxygenator aufweist.

19. Herz-Lungen-Maschine nach einem der Ansprüche 17 oder 18, **dadurch gekennzeichnet, dass** die Herz-Lungen-Maschine einen Dialysator aufweist.

20. Herz-Lungen-Maschine nach einem der Ansprüche 17 bis 19, **dadurch gekennzeichnet, dass** die Herz-Lungen-Maschine einen Filter aufweist.

21. Herz-Lungen-Maschine nach einem der Ansprüche 17 bis 20, **dadurch gekennzeichnet, dass** die Herz-Lungen-Maschine eine Gasversorgung aufweist, insbesondere eine Sauerstoffversorgung.

## Claims

1. Extracorporeal blood pump for aspiration and displacement of blood, wherein the blood pump has one or two blood chambers and a mechanical drive unit, each of the blood chambers having a membrane, a blood inlet channel and a blood outlet channel,
- wherein in the case of the blood pump with one blood chamber, the drive unit is adjacent to the blood chamber, wherein the drive unit has a gas inlet and a gas outlet as well as two pressure chambers, wherein each pressure chamber has an active surface, wherein a blood chamber is separated from a pressure chamber via a membrane, wherein the drive unit is actively connected to the position of the membrane, and wherein an area between the active surface and the membrane has a coupling rod which is designed to couple the movement of the active surface and the membrane to one another;
- wherein in the case of the blood pump with two blood chambers, the drive unit is arranged between the blood chambers, the blood outlet channels of both blood chambers are connected to one another and an area between the membranes has a coupling rod which is designed to couple the movement of the two membranes to one another, whereby the drive unit has a gas inlet and a gas outlet as well as a pressure chamber, wherein the pressure chamber is separated from a blood chamber via a membrane, wherein the drive unit is actively connected to the position of the membrane.

2. Extracorporeal blood pump according to claim 1, **characterised in that** the blood inlet channels of both blood chambers are connected to one another.

3. Extracorporeal blood pump according to one of claims 1 or 2, **characterised in that a** connecting area of the blood outlet channels has a return flow throttle.

4. Extracorporeal blood pump according to one of the preceding claims, **characterised in that** the drive unit has two pressure chambers, each pressure chamber being adjacent to a blood chamber via a membrane.

5. Extracorporeal blood pump according to one of the preceding claims, **characterised in that** a blood chamber has a rotationally symmetrical area.

6. Extracorporeal blood pump according to one of the preceding claims, **characterised in that** a blood inlet channel and a blood outlet channel are predominantly arranged in the circumferential direction in the rotationally symmetrical area of a blood chamber.

7. Extracorporeal blood pump according to one of the preceding claims, **characterised in that** a blood inlet channel has a return flow throttle.

8. Extracorporeal blood pump according to one of the preceding claims, **characterised in that** the drive unit has a gas inlet valve and a gas outlet valve as well as a switching device, wherein the gas inlet valve and the gas outlet valve have a closed position and an open position with respect to a gas flow path, wherein the switching device has two switching end states, in particular the switching device has exactly two switching states.

9. Extracorporeal blood pump according to claim 8, **characterised in that** a switching end state for a blood chamber has the gas inlet valve in the open position and the gas outlet valve in the closed position or the gas inlet valve in the closed position and the gas outlet valve in the open position.

10. Extracorporeal blood pump according to one of claims 8 or 9, **characterised in that** the switching device has a magnet.

11. Extracorporeal blood pump according to one of claims 8 to 10, **characterised in that** the switching device has a lever mechanism.

12. Extracorporeal blood pump according to one of claims 8 to 11, **characterised in that** the switching device has a gate to a spring-loaded roller.

13. Extracorporeal blood pump according to one of the preceding claims, **characterised in that** the coupling rod has a cavity inside.

14. Extracorporeal blood pump according to one of the preceding claims, **characterised in that** the coupling rod has a first component and a second component, wherein the first component and the second component are connected with a spring.

15. Extracorporeal blood pump according to one of the preceding claims, **characterised in that** the coupling rod has a valve which is designed to allow a designated gas flow to flow through the cavity in the coupling rod and then through the valve in the coupling rod and via a bypass out of a pressure chamber.

16. Extracorporeal blood pump according to claim 15, **characterised in that** the bypass has a closure device.

17. Heart-lung machine for pumping and processing blood, wherein the heart-lung machine has a blood supply and a blood discharge, **characterised in that** the heart-lung machine has a blood pump according to one of the preceding claims.

18. Heart-lung machine according to claim 17, **characterised in that** the heart-lung machine has an oxygenator.

19. Heart-lung machine according to one of claims 17 or 18, **characterised in that** the heart-lung machine has a dialyser.

20. Heart-lung machine according to one of claims 17 to 19, **characterised in that** the heart-lung machine has a filter.

21. Heart-lung machine according to one of claims 17 to 20, **characterised in that** the heart-lung machine has a gas supply, in particular an oxygen supply.

## Revendications

1. Pompe à sang extracorporelle pour aspirer et refouler le sang, la
pompe à sang comportant une ou deux chambres à sang et une unité d'entraînement mécanique, chacune des chambres à sang comportant une membrane, un canal d'entrée du sang et un canal de sortie du sang,
- dans le cas de la pompe à sang ayant une chambre à sang, l'unité d'entraînement est adjacente à la chambre à sang, l'unité d'entraînement comportant une entrée de gaz et une sortie de gaz ainsi que deux chambres de pression, chaque chambre de pression comportant une surface active, une chambre à sang étant séparée d'une chambre hyperbare par une membrane, l'unité d'entraînement étant en liaison fonctionnelle avec la position de la membrane, et une zone entre la surface active et la membrane comportant une tige d'accouplement conçue pour coupler le mouvement de la surface active et de la membrane ;
- dans le cas de la pompe à sang ayant deux chambres à sang, l'unité d'entraînement étant disposée entre les chambres à sang, les canaux de sortie du sang des deux chambres à sang étant reliés entre eux et une zone entre les membranes comportant une tige d'accouplement adaptée pour coupler le mouvement des deux membranes, et en ce que l'unité d'entraînement comporte une entrée de gaz et une sortie de gaz, ainsi qu'une chambre hyperbare, la chambre hyperbare étant séparée de la chambre à sang par une membrane, l'unité d'entraînement se trouvant en liaison active avec la position de la membrane.

2. Pompe à sang extracorporelle selon la revendication 1, **caractérisée en ce que** les canaux d'entrée du sang des deux chambres à sang sont reliés l'un à l'autre.

3. Pompe à sang extracorporelle selon l'une des revendications 1 ou 2, **caractérisée en ce qu'**une zone de connexion des canaux de sortie du sang comporte un étranglement de reflux.

4. Pompe à sang extracorporelle selon l'une quelconque des revendications précédentes, **caractérisée en ce que** l'unité d'entraînement comporte deux chambres hyperbares, chaque chambre hyperbare étant adjacente à une chambre à sang par l'intermédiaire d'une membrane.

5. Pompe à sang extracorporelle selon l'une quelconque des revendications précédentes, **caractérisée en ce qu'**une chambre à sang comporte une zone à symétrie de révolution.

6. Pompe à sang extracorporelle selon l'une quelconque des revendications précédentes, **caractérisée en ce qu'**un canal d'entrée du sang et un canal de sortie du sang sont disposés de manière prédominante dans la direction circonférentielle sur la zone à symétrie de révolution d'une chambre à sang.

7. Pompe à sang extracorporelle selon l'une quelconque des revendications précédentes, **caractérisée en ce qu'**un canal d'entrée de sang comporte un étranglement de reflux.

8. Pompe à sang extracorporelle selon l'une quelconque des revendications précédentes, **caractérisée en ce que** l'unité d'entraînement comporte une vanne d'entrée de gaz et une vanne de sortie de gaz ainsi qu'un dispositif de commutation, la vanne d'entrée de gaz et la vanne de sortie de gaz comportant une position de fermeture et une position d'ouverture par rapport à un trajet d'écoulement de gaz, le dispositif de commutation comportant deux états finaux de commutation, notamment, le dispositif de commutation comportant exactement deux états de commutation.

9. Pompe à sang extracorporelle selon la revendication 8, **caractérisée en ce qu'**un état final de commutation pour une chambre à sang comporte la vanne d'entrée du gaz en position d'ouverture et la vanne de sortie du gaz en position de fermeture et la vanne de sortie du gaz en position d'ouverture.

10. Pompe à sang extracorporelle selon l'une des revendications 8 ou 9, **caractérisée en ce que** le dispositif de commutation comporte un aimant.

11. Pompe à sang extracorporelle selon l'une des revendications 8 à 10, **caractérisée en ce que** le dispositif de commutation comporte un réducteur à levier.

12. Pompe à sang extracorporelle selon l'une des revendications 8 à 11, **caractérisée en ce que** le dispositif de commutation comporte une coulisse avec un rouleau à ressort.

13. Pompe à sang extracorporelle selon l'une quelconque des revendications précédentes, **caractérisée en ce que** la tige d'accouplement comporte une cavité à l'intérieur.

14. Pompe à sang extracorporelle selon l'une quelconque des revendications précédentes, **caractérisée en ce que** la tige d'accouplement comporte un premier composant et un deuxième composant, le premier composant et le deuxième composant étant reliés par un ressort.

15. Pompe à sang extracorporelle selon l'une quelconque des revendications précédentes, **caractérisée en ce que** la tige d'accouplement comporte une vanne adaptée pour permettre à un flux de gaz désigné de s'écouler à travers la cavité dans la tige d'accouplement et, par la suite, à travers la vanne dans la tige d'accouplement et à travers une dérivation à partir d'une chambre hyperbare.

16. Pompe à sang extracorporelle selon la revendication 15, **caractérisée en ce que** la dérivation comporte un dispositif d'obturation.

17. Machine cœur-poumon pour transporter et traiter le sang, la machine cœur-poumon comportant une amenée de sang et une évacuation de sang, **caractérisée en ce que** la machine cœur-poumon comporte une pompe à sang selon l'une des revendications précédentes.

18. Machine cœur-poumon selon la revendication 17, **caractérisée en ce que** la machine cœur-poumon comporte un générateur d'oxygène.

19. Machine cœur-poumon selon l'une des revendications 17 ou 18, **caractérisée en ce que** la machine cœur-poumon comporte un hémodyaliseur.

20. Machine cœur-poumon selon l'une des revendications 17 à 19, **caractérisée en ce que** la machine cœur-poumon comporte un filtre.

21. Machine cœur-poumon selon l'une des revendications 17 à 20, **caractérisée en ce que** la machine cœur-poumon comporte une alimentation en gaz, notamment une alimentation en oxygène.
